# EUROPEAN PATENT SPECIFICATION

(11) **EP 4 053 095 B1**
(45) Date of publication and mention of the grant of the patent: **10.07.2024**
(21) Application number: 20881314.7
(22) Date of filing: 29.10.2020
(51) Int. Cl.: C07C 37/66, C07C 39/235, C07F 9/145

(54) **METHOD FOR PRODUCING SODIUM SALT OF PHENOLIC COMPOUND AND BISPHOSPHITE COMPOUND**
VERFAHREN ZUR HERSTELLUNG VON NATRIUMSALZ EINER PHENOLVERBINDUNG UND BISPHOSPHITVERBINDUNG
PROCÉDÉ DE PRODUCTION DE SEL DE SODIUM DE COMPOSÉ PHÉNOLIQUE ET COMPOSÉ BISPHOSPHITE

(30) Priority: 31.10.2019 JP 2019198915; 20.05.2020 JP 2020088188
(43) Date of publication of application: 07.09.2022
(73) Proprietor: Mitsubishi Chemical Corporation, Chiyoda-ku Tokyo 100-8251 (JP)
(72) Inventor: SATO Takashi, Tokyo 100-8251 (JP); KOJYOU Atsushi, Tokyo 100-8251 (JP); TANAKA Yoshiyuki, Tokyo 100-8251 (JP)
(74) Representative: Hoffmann Eitle
(86) International application number: PCT/JP2020/040693
(87) International publication number: WO 2021/085557

(56) References cited:
- WO-A1-2008/000727
- WO-A1-2019/203095
- WO-A2-2004/076464
- JP-A- 2002 177 763
- JP-A- H1 045 775
- JP-A- H10 110 205
- US-B1- 6 610 891

## Description

### TECHNICAL FIELD

The present invention relates to a method for producing a sodium salt of a phenol compound, and more particularly, to a method for producing a corresponding sodium salt in a short time with a high yield by allowing to react a phenol compound having two or more phenolic hydroxyl groups with sodium to smoothly substitute almost all the phenolic hydroxyl groups with sodium, and to a method for producing a bisphosphite compound.

### BACKGROUND ART

As a method for producing a sodium salt of a phenol compound, a method of allowing a phenol compound to react with metallic sodium is known (Non-Patent Literature 1).

In Non-Patent Literature 1, metallic sodium is finely added to a mixture of phenol and ether, and the mixture is stirred and mixed to produce sodium phenoxide.

Patent Literature 1 describes an optically active monodentate phosphate and/or phosphoramidite having an optically active biphenol moiety with axial chirality, and a process for producing an optically active compound. The process comprises the transformation of a prochiral compound in the presence of a chiral transition metal catalyst, wherein the transition metal catalyst comprises a reaction mixture of a transition metal belonging to the groups 4 to 12 of the periodic table, or a compound thereof, and the optically active monodentate phosphite and/or phosphoramidite as an optically active ligand.

Patent Literature 2 describes the use of transition metal-carbene complexes, in which the carbene ligand(s) is bonded to the transition metal exclusively via carbene carbon atoms, in organic light-emitting diodes, to organic light-emitting diodes comprising at least one aforementioned transition metal-carbene complex, to a light-emitting layer comprising at least one aforementioned transition metal-carbene complex, to organic light-emitting diodes comprising at least one specific light-emitting layer, and to devices which comprise at least one specific organic light-emitting diode. In example A) Patent Literature 2 describes the preparation of the di-sodium salt of 2,2'-dihydroxy-biphenyl using metallic sodium.

### CITATION LIST

### NON-PATENT LITERATURE

Non-Patent Literature 1: Journal of the Chemical Society of Japan, 1980, (5), p. 733 to 737 "Selective ortho-Alkylation Reaction of Phenols Using Metallic Sodium"

### PATENT LITERATURE

Patent Literature 1: WO 2004/076464 A2
Patent Literature 2: WO 2008/000727 A1

### SUMMARY OF INVENTION

### TECHNICAL PROBLEM

Conventional methods in which metallic sodium is added to a phenol compound and allowing to be reacted have the following problems.
(1) It takes a long time to complete the reaction.
(2) When a phenol compound having a plurality of reaction points, namely, a phenol compound having two or more phenolic hydroxyl groups is used as a substrate, it is difficult to substitute almost all of the phenolic hydroxyl groups with sodium, and a yield of a target product is low.
(3) Since activity of metallic sodium is low, it is necessary to add sodium excessively to the substrate, and unreacted sodium needs to be separated by filtration or the like after the reaction. In addition, a lot of labor is required for treating the active sodium after separation.

An object of the present invention is to solve the problems of the conventional methods and to provide a method for efficiently producing a target sodium salt from a phenol compound having two or more phenolic hydroxyl groups in a short time and at a high yield.

### SOLUTION TO PROBLEM

As a result of repeated studies to solve the above problems, a present inventor has found that when a sodium dispersion liquid (sodium dispersion) in which sodium particles are dispersed is used for substituting a phenol compound having two or more phenolic hydroxyl groups, sodium is consumed in a reaction before precipitation of the sodium particles, the reaction efficiently proceeds in a short time, and the phenol compound in which almost all phenolic hydroxyl groups are substituted by sodium can be obtained at a high yield.

In the past, it has been considered that when a solution in which a substrate is dissolved in a solvent and the sodium dispersion liquid in which the sodium particles are dispersed are mixed, the sodium particles precipitate after mixing due to a difference in specific gravity between sodium and the solvent, and it is difficult to complete the substitution reaction by sodium. Therefore, use of the sodium dispersion liquid in such a reaction has not been expected.

Namely, the present invention is defined in the appended claims.

### EFFECTS OF INVENTION

According to the present invention, a sodium salt of a phenol compound can be produced in a much shorter time than the conventional method by a simple and safe operation of mixing a sodium dispersion liquid in which sodium particles are dispersed and a phenol compound solution. In addition, almost all of the phenolic hydroxyl groups can be smoothly substituted by sodium by using a phenol compound having two or more phenolic hydroxyl groups as a substrate, so that the yield of the target product is high.

Since high substitution activity with sodium can be obtained, it is sufficient to use sodium in an amount substantially equivalent to the substrate, and labor required for treatment of unreacted sodium is significantly reduced.

### BRIEF DESCRIPTION OF DRAWINGS

[Fig. 1] Fig. 1 is a graph showing results of Example 1.
[Fig. 2] Fig. 2 is a graph showing results of Comparative Example 1.

### DESCRIPTION OF EMBODIMENTS

Hereinafter, the present invention will be described in detail, whereas the present invention is not limited to the following description, and can be optionally modified and implemented without departing from the scope of the present invention defined by the claims. In the present specification, when a numerical value or a physical property value is described before and after the expression "to", the expression "to" is used as the meaning of including the values before and after that.

The present invention relates to a method for producing a sodium salt of a phenol compound, including a step of mixing a phenol compound solution in which a phenol compound having two or more phenolic hydroxyl groups (hereinafter, sometimes referred to as a "raw material phenol compound") is dissolved with a sodium dispersion liquid in which sodium particles are dispersed.

### <Raw Material Phenol Compound>

The raw material phenol compound as a reaction substrate in the method for producing the sodium salt of the phenol compound according to the present embodiment is a phenol compound having two or more phenolic hydroxyl groups. The number of the phenolic hydroxyl groups in the phenol compound may be two or more, and an upper limit of the number of phenolic hydroxyl groups is not particularly limited, and is usually 14 or less.

In the case of a phenol compound having one phenolic hydroxyl group, a sodium salt can be produced in a relatively short time by a conventional method even without using the method according to the present embodiment.

In the present embodiment, a phenol compound having two or more phenolic hydroxyl groups, which requires a long time for the reaction and cannot completely push the reaction in the conventional method, is used as the raw material phenol compound.

In the present embodiment, the phenol compound is a phenol compound in a broad sense including all compounds having a benzene ring and a phenolic hydroxyl group. A ring serving as a matrix to which the phenolic hydroxyl group is bound has at least one benzene ring, and may be a condensed ring such as a naphthalene ring, or may be a polycyclic compound such as biphenyl or terphenyl wherein a benzene ring is bound by a single bond or any linking group.

Although not particularly limited, examples of the raw material phenol compound used in the present embodiment include dihydroxybenzene, dihydroxynaphthalene, dihydroxybiphenyl, dihydroxyterphenyl, trihydroxyphenol, and tetrahydroxyphenol. Among these, dihydroxybiphenyl is preferable, and dihydroxybiphenyl of the following Formula (1) is particularly preferable, since it is useful as a raw material of bisphosphite which can be suitably used as a ligand of a catalyst.

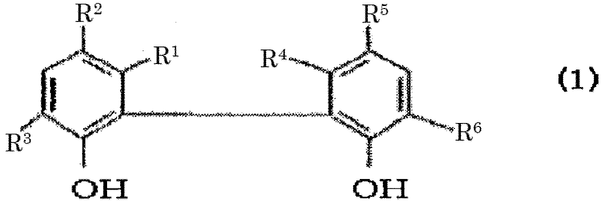

In the formula, each of R¹ and R⁴ independently represents a hydrogen atom, an alkyl group having 1 to 12 carbon atoms, a cycloalkyl group having from 3 to 12 carbon atoms, an alkoxy group having from 1 to 12 carbon atoms, a silyl group having from 1 to 12 carbon atoms, a siloxy group having from 1 to 12 carbon atoms, or a halogen atom, and R², R³, R⁵, and R⁶ each independently represents an alkyl group having from 1 to 20 carbon atoms, a cycloalkyl group having from 3 to 20 carbon atoms, an alkoxy group having from 1 to 20 carbon atoms, an aryl group having from 6 to 20 carbon atoms, a silyl group having from 1 to 20 carbon atoms, or a siloxy group having from 1 to 20 carbon atoms.

In Formula (1), each of R¹ and R⁴ is preferably a lower alkyl group having from 1 to 3 carbon atoms such as a methyl group or an ethyl group, or a lower alkoxy group such as a methoxy group or an ethoxy group, and particularly preferably a methyl group or a methoxy group.

Examples of R², R³, R⁵, and R⁶ include a linear or branched alkyl group having from 1 to 20 carbon atoms, such as a methyl group, an ethyl group, a propyl group, an isopropyl group, a butyl group, a tertiary butyl group, a tertiary pentyl group, a tertiary hexyl group, a nonyl group, and a decyl group; a cycloalkyl group having from 3 to 20 carbon atoms, such as a cyclopropyl group, a cyclohexyl group, and a cyclooctyl group; an alkoxy group having from 1 to 20 carbon atoms, such as a methoxy group, an ethoxy group, and a tertiary butoxy group; an aryl group having from 6 to 20 carbon atoms, such as a phenyl group and a p-tolyl group; a silyl group having from 1 to 20 carbon atoms, such as a trimethylsilyl group; and a siloxy group having from 1 to 20 carbon atoms, such as a trimethylsiloxy group. Among these, the branched alkyl group or cycloalkyl group such as an isopropyl group, a tertiary butyl group, a tertiary pentyl group, a tertiary hexyl group, a cyclopropyl group, and a cyclohexyl group is preferable.

Among these, the dihydroxybiphenyl represented by Formula (1) is preferably 2,2'-biphenol having a tertiary butyl group as a substituent. Examples thereof include 3,3',5,5'-tetratertiary butyl-2,2'-biphenol and 3,3',5,5'-tetratertiary butyl-6,6'-dimethyl-2,2'-biphenol.

### <Phenol Compound Solution>

The phenol compound solution used in the present embodiment can be prepared by mixing the raw material phenol compound and a solvent. At this time, the raw material phenol compound is preferably completely dissolved in the solvent in advance. However, for example, the raw material phenol compound may be dissolved in the solvent at the time of mixing with the sodium dispersion liquid described later or at the time of reaction with the sodium dispersion liquid. The phenol compound solution can be prepared in air or in an inert gas atmosphere such as nitrogen.

The solvent used in the phenol compound solution may be any solvent as long as it does not have reactivity with the raw material phenol compound, and is not particularly limited as long as it is one or more of a non-polar solvent and/or a polar solvent.

From a viewpoint of solubility of the raw material phenol compound, at least one solvent is preferably the polar solvent. Examples of the polar solvent include polar organic solvents such as ketones, ethers, and esters.

Examples of the ketones include lower alkyl ketones having from 1 to 10 carbon atoms, such as dimethyl ketone, diethyl ketone, methyl ethyl ketone, methyl n-propyl ketone, methyl isopropyl ketone, methyl n-butyl ketone, methyl isobutyl ketone, methyl n-pentyl ketone, methyl isopentyl ketone, ethyl n-propyl ketone, ethyl isopropyl ketone, ethyl n-butyl ketone, and ethyl isobutyl ketone; and cyclic ketones having from 3 to 10 carbon atoms, such as cyclohexanone and cyclopentanone.

Examples of the ethers include lower alkyl ethers having from 2 to 10 carbon atoms, such as dimethyl ether, diethyl ether, methyl ethyl ether, methyl n-propyl ether, methyl isopropyl ether, methyl n-butyl ether, methyl isobutyl ether, methyl n-pentyl ether, methyl isopentyl ether, ethyl n-propyl ether, ethyl isopropyl ether, ethyl n-butyl ether, ethyl isobutyl ether, dimethoxyethane, and diethoxyethane; cyclic ethers such as tetrahydrofuran; and diaryl ethers such as diphenyl ether.

Examples of the esters include lower aliphatic carboxylic acid esters such as alkyl formic acid esters, alkyl acetic acid esters, and alkyl propionic acid esters; alkyl carbonic acid diesters such as dimethyl carbonic acid esters, diethyl carbonic acid esters, methylethyl carbonic acid esters, and dibutyl carbonic acid esters; oxalic acid diesters such as dimethyl oxalic acid esters and diethyl oxalic acid esters; and acetic acid esters such as ethyl acetate, propyl acetate, butyl acetate, ethylene glycol acetic acid esters, and fatty acid esters of ethylene glycol.

Among these, ethers are preferable, cyclic ethers are more preferable, and tetrahydrofuran is particularly preferable in terms of availability, ease of handling, and the like.

A concentration of the phenol compound solution depends on the solubility in the solvent to be used, but is not particularly limited. For example, the concentration is about 0.1 to 1 mol/L. When the concentration is at the lower limit or more, reaction efficiency is more excellent. When the concentration is equal to or less than the upper limit, it is possible to prevent necessity of methods such as heat removal required by increase in a reaction speed, and at the same time, increase in a temperature in a system.

### <Sodium Dispersion Liquid>

The sodium dispersion liquid used in the present embodiment is a dispersion liquid in which the sodium particles are dispersed in the solvent. The term "dispersion" refers to a state in which particles insoluble in the solvent are uniformly present in the solvent without being precipitated, and in the present invention, refers to a state in which the sodium particles are not precipitated even when the sodium particles are allowed to stand in the solvent for one week or more.

As the solvent, an organic solvent can be generally selected, and any of a nonpolar solvent and a non-polar solvent may be used, but a hydrocarbon solvent is preferable. Examples of the hydrocarbon solvent include trans-oil, kerosene, paraffin, hexane, toluene, and dodecane. One of the above components may be used alone, or two or more of the components may be used in combination.

Among these, paraffin is preferably used in terms of availability, ease of handling, and the like.

Examples of the method for preparing the sodium dispersion liquid include a method in which metallic sodium is heated and melted in an organic solvent and stirred and dispersed using a high-speed stirring device, a stick mixer, a homomixer, a homogenizer, a rotor-rotating shearing machine, or a combination thereof (hereinafter, the machines may be referred to as a "stirring machine"), and a method in which a sodium melt and an organic solvent heated to a temperature equal to or higher than a melting point (about 98°C) of sodium are stirred and dispersed using the stirring machine. For example, methods known in the related art described in JP-A-H09-209006, JP-A-H10-110205, JP-A-2004-300577, JP-A-2006-218398, JP-A-2007-224360, and the like can be used.

A heating temperature during stirring and dispersion is preferably equal to or lower than a boiling point of the organic solvent used.

In the present embodiment, the sodium dispersion liquid is preferably prepared by a method including the following first and second steps.
(1) First step: a step of preparing a high-concentration sodium dispersion liquid having a sodium concentration of about 20 to 50 weight% by stirring and dispersing the sodium melt in a first solvent using the above-described stirring machine.
(2) Second step: Next, a step of preparing a sodium dispersion liquid having a sodium concentration of about 5 to 20 weight% by further adding a second solvent to the high-concentration sodium dispersion liquid and mixing the high-concentration sodium dispersion liquid with the second solvent.

In the first step, a temperature condition for stirring and dispersing the sodium melt in the first solvent is preferably a temperature equal to or higher than the melting point of sodium and equal to or lower than the boiling point of the first solvent. In the second step, the temperature condition for mixing the second solvent is preferably, for example, a temperature equal to or higher than the melting point of sodium and equal to or lower than the boiling points of the first and second solvents.

Here, the first solvent is preferably the hydrocarbon solvent. As the second solvent, the hydrocarbon solvent or the polar solvent can be used. As the polar solvent, the same polar solvents as those described in the case of dissolving the raw material phenol compound can be used. The second solvent is preferably the same solvent as the first solvent or the same solvent as that used in the preparation of the phenol compound solution from the viewpoint that the solvent can be easily recovered and reused from a reaction mixture after the reaction. The second solvent is preferably an ether, more preferably tetrahydrofuran.

In the second step, it is preferable to adjust a sodium dispersion liquid to have a sodium concentration of preferably about 10 to 20 weight% from the viewpoint of the dispersibility of the sodium particles and the reactivity with the raw material phenol compound.

An atmosphere at the time of preparing the sodium dispersion liquid is preferably an atmosphere in which deterioration of the finally obtained sodium dispersion liquid does not occur, and is preferably, for example, an inert gas atmosphere such as nitrogen.

An average particle diameter (Median diameter D₅₀) of the sodium particles in the finally prepared sodium dispersion liquid is preferably 70 µm or less, more preferably 1 to 70 µm, still more preferably 1 to 30 µm, and yet still more preferably 1 to 10 µm. Such fine sodium particles do not precipitate in the solvent for a long time, and are uniformly present. Since the reaction activity is high, substitution of the phenolic hydroxyl group with sodium can be performed in a shorter time.

The average particle diameter (Median diameter D₅₀) of the sodium particles in the sodium dispersion liquid can be adjusted by the number of revolutions, rotation speed, stirring time, and the like of the stirring machine. The average particle diameter (Median diameter D₅₀) of the sodium particles can be determined using a laser diffraction-type particle size distribution measurement apparatus, an optical microscope (fluorescence microscope), or the like.

### <Mixing and Reaction of Sodium Dispersion Liquid and Phenol Compound Solution>

In the present embodiment, by mixing the sodium dispersion liquid prepared as described above with the phenol compound solution, the phenolic hydroxyl group of the raw material phenol compound is allowed to react with sodium, thereby producing a sodium salt.

### (Mixing)

The mixing is preferably performed under such conditions that the sodium particles do not associate with each other in the mixed liquid, and examples of the method therefor include the following mixing methods (1) to (3). One of the methods may be used, or two or more of the methods may be used in combination.
(1) The sodium dispersion liquid and the phenol compound solution are mixed at a temperature lower than the melting point of sodium.
(2) With stirring the sodium dispersion liquid, the phenol compound solution is added little by little.
(3) With stirring the phenol compound solution, the sodium dispersion liquid is added little by little.

Among these, the method of mixing the sodium dispersion liquid and the phenol compound solution at the temperature lower than the melting point of sodium as described in (1) above is particularly preferable because the association of the sodium particles can be sufficiently prevented. When the average particle diameter (Median diameter D₅₀) of the sodium particles is 70 µm or less, the sodium particles tend to easily associate when the sodium dispersion liquid is mixed with the phenol compound solution. When the sodium particles are associated with each other in the mixed liquid, the reaction activity is reduced, and a larger amount of sodium than an equivalent amount with respect to the substrate may be required. Such a problem can be prevented by mixing the two liquids at a temperature lower than the melting point of sodium.

Further, the mixing is preferably performed at a temperature lower than the melting point of sodium and lower than a lowest boiling point among the boiling points of the solvents contained in the sodium dispersion liquid and the phenol compound solution.

A lower limit of a mixing temperature is not particularly limited, but is preferably a temperature at which the raw material phenol compound is not precipitated. Further, in the case of industrially producing the sodium salt, as described in the above (3), from the viewpoint of safety, it is preferable to use the method of adding the sodium dispersion liquid little by little with stirring of the phenol compound solution in combination.

The mixing of both liquids may be performed in air or in the inert gas atmosphere such as nitrogen.

A mixing ratio of the sodium dispersion liquid and the phenol compound solution may be such that an amount of sodium in the sodium dispersion liquid is equal to or greater than the reaction equivalent of the phenolic hydroxyl group of the raw material phenol compound in the phenol compound solution.

In the present embodiment, since the sodium dispersion liquid is used, high reaction activity can be obtained, and it is not necessary to use sodium which is much excessive with respect to the reaction equivalent. In general, the sodium dispersion liquid and the phenol compound solution may be mixed so that the amount of sodium is about 1 to 1.5 equivalent times, preferably about 1 to 1.1 equivalent times, more preferably about 1 to 1.05 equivalent times, and particularly preferably about 1 to 1.01 equivalent times the amount of the phenolic hydroxyl group of the raw material phenol compound.

After mixing a predetermined amount of the sodium dispersion liquid and the phenol compound solution, the temperature is raised to a reaction temperature and the reaction is performed for a predetermined time. As described above, the reaction temperature is preferably lower than the melting point of sodium. More preferably, the reaction temperature is lower than the melting point of sodium and lower than the boiling points of the solvents used in the sodium dispersion liquid and the phenol compound solution, specifically, the organic solvent and the polar solvent.

When the temperature is equal to or higher than the melting point of sodium, sodium tends to be associated and an association tends to be easily formed. When the temperature is higher than the boiling point of the organic solvent or polar solvent used in the sodium dispersion liquid or the phenol compound solution, there is a tendency that good dispersibility of sodium cannot be maintained due to the boiling solvent.

A lower limit of the reaction temperature is not particularly limited, but is preferably a temperature at which the raw material phenol compound is not precipitated.

The reaction may be performed in air or in the inert gas atmosphere such as nitrogen, but is preferably performed in the inert gas atmosphere such as nitrogen in order to reduce loss due to oxidation of the raw material phenol compound.

A reaction time, that is, a time for which both liquids are mixed and then held at the reaction temperature varies depending on the reaction temperature, reactivity of the raw material phenol compound for substitution with sodium, and an amount of the raw material phenol compound and sodium to be reacted, but is usually about 1 to 5 hours.

### (Sodium Particles in Mixed Liquid of Sodium Dispersion Liquid and Phenol Compound Solution)

When a large number of large sodium particles are present in the mixed liquid of the phenol compound solution and the sodium dispersion liquid, the dispersibility of the sodium particles and the reaction activity for substitution with sodium tend to be deteriorated. Therefore, the number of sodium particles having a major axis of more than 70 µm (hereinafter, may be referred to as "coarse particles") present in the mixed liquid is preferably 5 particles/mL or less, and particularly preferably 1 particle/mL or less.

Here, the major diameter corresponds to a maximum diameter of the sodium particles, namely, a particle diameter of a portion where an interval between plates is the largest when the sodium particles are sandwiched between two parallel plates.

In order to set the number of particles having the major axis of more than 70 µm to be equal to or less than the upper limit, it is preferable to mix both liquids under a condition in which the sodium particles do not associate with each other in the mixed liquid. As a method therefor, for example, the mixing method from (1) to (3) described above is exemplified.

The number of sodium particles having the major axis of more than 70 µm may be measured by a method known in the related art, and can be measured using, for example, AccuSizer A9000 series manufactured by Particle Sizing Systems.

### <Use of Sodium Salt of Phenol Compound>

The sodium salt of the phenol compound produced by the method for producing the sodium salt of the phenol compound according to the present embodiment is industrially useful as a precursor or the like of many organic compounds.

A derivative of a phosphite compound can be produced by further allowing the sodium salt of the phenol compound as a precursor to react with a phosphorus compound, and the derivatives can be used as a ligand of a catalyst, an intermediate of a pharmaceutical product, or the like.

For example, a bisphosphite compound can be produced by allowing a sodium salt of dihydroxybiphenyl produced by the method for producing the sodium salt according to the present embodiment to react with a phosphorus compound. Examples of the dihydroxybiphenyl include a compound represented by Formula (1) described above. Examples of the corresponding bisphosphite compound include a compound represented by Formula (2).

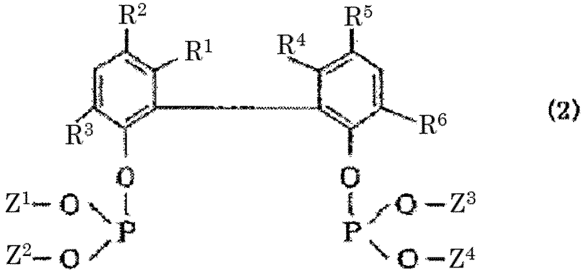

In the formula, each of R¹ and R⁴ independently represents a hydrogen atom, an alkyl group having from 1 to 12 carbon atoms, a cycloalkyl group having from 3 to 12 carbon atoms, an alkoxy group having from 1 to 12 carbon atoms, a silyl group having from 1 to 12 carbon atoms, a siloxy group having from 1 to 12 carbon atoms, or a halogen atom, each of R², R³, R⁵, and R⁶ independently represents an alkyl group having from 1 to 20 carbon atoms, a cycloalkyl group having from 3 to 20 carbon atoms, an alkoxy group having from 1 to 20 carbon atoms, an aryl group having from 6 to 20 carbon atoms, a silyl group having from 1 to 20 carbon atoms, or a siloxy group having from 1 to 20 carbon atoms, and Z¹, Z², Z³, and Z⁴ each independently represents an alkyl group having from 1 to 20 carbon atoms, a cycloalkyl group having from 3 to 20 carbon atoms, a substituted or unsubstituted aryl group having from 6 to 20 carbon atoms, an aralkyl group having from 7 to 20 carbon atoms, or a heteroaryl group having from 1 to 20 carbon atoms.

In Formula (1), examples of Z¹, Z², Z³, and Z⁴ include a linear or branched alkyl group having from 1 to 20 carbon atoms such as a methyl group, an ethyl group, a propyl group, an isopropyl group, a butyl group, a sec-butyl group, a tertiary butyl group, an octyl group, a nonyl group, and a dodecyl group; a cycloalkyl group having from 3 to 20 carbon atoms such as a cyclopropyl group, a cyclohexyl group, and a cyclooctyl group; an aryl group having from 6 to 20 carbon atoms which may have a substituent such as a phenyl group, an α-naphthyl group, a β-naphthyl group, a methoxyphenyl group, a dimethoxyphenyl group, a carbomethoxyphenyl group, a nitrophenyl group, a cyanophenyl group, a chlorophenyl group, a dichlorophenyl group, a fluorophenyl group, a difluorophenyl group, a pentafluorophenyl group, a tolyl group, an ethylphenyl group, a nonylphenyl group, a trifluoromethylphenyl group, a perfluorobutylphenyl group, a methylnaphthyl group, a methoxynaphthyl group, a chloronaphthyl group, a nitronaphthyl group, and a tetrahydronaphthyl group; and an aralkyl group having from 7 to 20 carbon atoms such as a benzyl group. Examples of Z¹, Z², Z³, and Z⁴ also include a heteroaryl group having from 1 to 20 carbon atoms, which is an aromatic group containing a hetero element. Examples thereof include a 2-pyridyl group, a 3-pyridyl group, a 4-pyridyl group, a 4-methyl-2-pyridyl group, a 6-methyl-2-pyridyl group, a 3-nitro-2-pyridyl group, a 2-pyrazyl group, a 4-pyrimidyl group, a 4-methyl-2-pyrimidyl group, a 4-benzofuryl group, a 5-benzofuryl group, a 5-benzothienyl group, a 2-quinolyl group, a 4-quinolyl group, a 6-quinolyl group, an 8-quinolyl group, a 5-nitro-8-quinolyl group, a 1-isoquinolyl group, a 3-isoquinolyl group, a 5-isoquinolyl group, a 2-quinoxalyl group, an 8-quinaldyl group, a 4-quinazolyl group, a 1-methyl-2-benzimidazolyl group, a 2-benzothiazolyl group, an N-methyl-2-carbazolyl group, a 2-benzofuranyl group, an N-methyl-4-indolyl group, an N-methyl-5-indolyl group, and a 4-methoxy-9-acridinyl group.

Further, a bisphosphite compound represented by the following Formula (3) can be produced by using 3,3',5,5'-tetratertiary-butyl-6,6'-dimethyl-2,2'-biphenol as the compound represented by the above-described Formula (1) and allowing a sodium salt of biphenyl produced by the production method according to the present embodiment to react with a phosphorus compound.

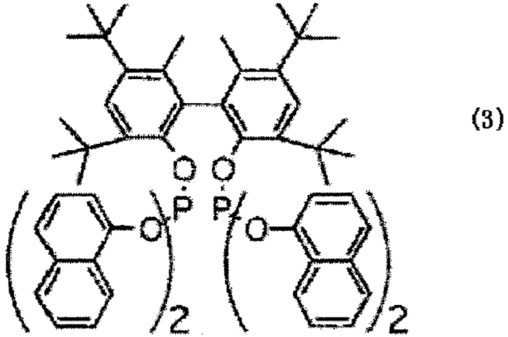

### EXAMPLES

Although the content of the present invention is further specifically explained using Examples, the present invention is not limited to the following Examples as long as the scope of the present invention defined by the claims is not exceeded. Values of various manufacturing conditions and evaluation results in the following Examples mean preferable values of an upper limit or a lower limit in the embodiments of the present invention, and a preferable range may be a range defined by a combination of the above-described upper limit or the above-described lower limit and the values of the following Examples or a combination of the values of Examples.

### [Various Measurement and Analysis Method]

In the following Examples and Comparative Examples, analysis of an average particle diameter of the sodium particles, and analysis of a product, and the like were performed by the following methods.

### <Average Particle Diameter of Sodium Particles in Sodium Dispersion Liquid>

An average particle diameter (Median diameter D₅₀) of the sodium particles in the sodium dispersion liquid was determined by a laser diffraction-type particle size distribution measurement apparatus SALD-2300 manufactured by SHIMADZU Corporation.

### <Number of Coarse Particles in Mixed Liquid>

The number of coarse particles having the major axis of more than 70 µm among the sodium particles in the mixed liquid was counted by the laser diffraction-type particle size distribution measurement apparatus SALD-2300 manufactured by SHIMADZU Corporation, and the number of coarse particles in 1 mL of the mixed liquid was obtained.

### <Analysis of Product>

A reaction product liquid after a lapse of a predetermined reaction time was analyzed by liquid chromatography.

In the following Examples and Comparative Examples, in order to enable analysis by liquid chromatography, a sodium salt (-ONa) was prepared, and then the sodium salt (-ONa) was further allowed to react with dichlorodimethylsilane (Si(CH₃)₂Cl₂) to prepare a dimethylsilyl ether compound, and the dimethylsilyl ether compound was subjected to liquid chromatography analysis.

A ratio (weight%) of each product was calculated from an area normalization of an analysis chart obtained by the liquid chromatography analysis, and used as selectivity of substitution by Na.

Namely, a raw material phenol compound having no silyl ether group at all was defined as an unreacted product, and a compound having one silyl ether group and one phenolic hydroxyl group, that is, a compound in which only one of the two phenolic hydroxyl groups was substituted by sodium was defined as a 1Na compound, and a compound having two silyl ether groups, that is, a compound in which all of the two phenolic hydroxyl groups were substituted by sodium was defined as a 2Na compound.

### <Raw Material Phenol Compound>

In the following Examples and Comparative Examples, 3,3',5,5'-tetratertiary-butyl-6,6'-dimethyl-2,2'-biphenol, which is a phenol compound having two phenolic hydroxyl groups, was used as a raw material phenol compound.

### [Example 1]

### <Preparation of Phenol Compound Solution>

The raw material phenol compound was dissolved in tetrahydrofuran (boiling point: about 66°C) at 25°C to prepare a phenol compound solution 1 having a concentration of 0.345 mol/L.

### <Preparation of Sodium Dispersion Liquid>

To 3.05 mL of a sodium dispersion 30 (average particle diameter of sodium: 10 µm, solvent: paraffin, concentration: 26 weight%) manufactured by KOBELCO ECO-SOLUTIONS CO., LTD., 45 mL of tetrahydrofuran was added at 25°C and stirred to prepare a sodium dispersion liquid 1 having a sodium concentration of 0.690 mol/L.

### <Substitution by Sodium and Reaction to Produce Dimethylsilyl Compound>

The sodium dispersion liquid 1 (48.05 mL) was placed in a four-necked flask and cooled to 2°C, and the phenol compound solution 1 (48.05 mL) was added dropwise to the sodium dispersion liquid 1 for 2 minutes with stirring the sodium dispersion liquid 1 for mixing. After completion of the dropwise addition, the temperature of the mixed liquid in the four-necked flask was increased at 3°C/min to 65°C, and the temperature was maintained at 65°C for 2 hours to perform a substitution reaction with sodium.

The mixing and reaction were performed in a nitrogen atmosphere.

The sodium dispersion liquid 1 and the phenol compound solution 1 used in the reaction are in amounts such that the amount of sodium is 1.00 equivalent with respect to 1 equivalent of the phenolic hydroxyl group of the raw material phenol compound.

When the liquid chromatography analysis was performed on the mixed liquid at the end of dropwise addition, results were shown in Table 1 (reaction time: 0 hour).

After reaction for 2 hours, dichlorodimethylsilane and tetrahydrofuran were mixed with the obtained reaction product liquid so that the concentration of dichlorodimethylsilane was 0.75 mol/L, and the obtained mixed liquid was added so that the amount of dichlorodimethylsilane was 5 equivalents times the reaction substrate, and the reaction was carried out for 10 minutes under stirring in the nitrogen atmosphere to perform a reaction to produce a dimethylsilyl compound.

The obtained reaction product was subjected to the liquid chromatography analysis, and the results shown in Table 1 (reaction time: 2 hours) were obtained.

In Example 1, the average particle diameter (Median diameter D₅₀) of the sodium particles in the prepared sodium dispersion liquid 1 was 10 µm. The number of coarse particles in the mixed liquid of the sodium dispersion liquid 1 and the phenol compound solution 1 was 0.

### < Substitution by Sodium and Reaction to Produce Dimethylsilyl Compound (Reaction Time: 0.5 Or 1 Hour)>

The Substitution by sodium and the reaction to produce a dimethylsilyl compound were performed in the same manner as described above except that the reaction time for the substitution by sodium was changed from 2 hours to 0.5 hours or 1 hour. The results of the liquid chromatography analysis at each reaction time are shown in Table 1 (reaction time: 0.5 hours) or Table 1 (reaction time: 1 hour).

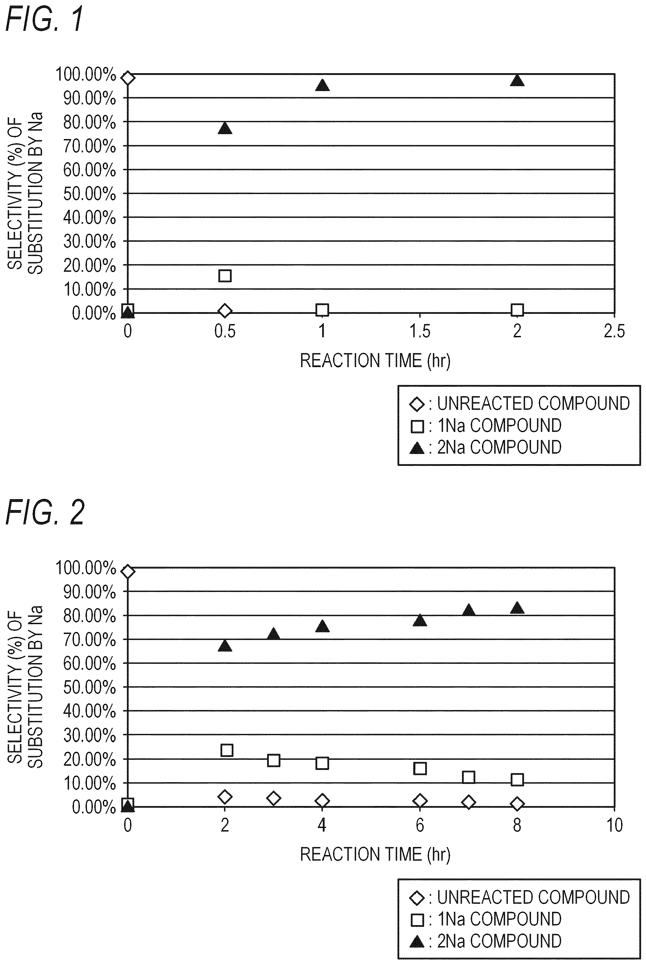

A relationship between the selectivity of substitution by Na and the reaction time based on the liquid chromatography analysis is shown in Fig. 1.

**Table 1**

| | | Reaction time (hr) | | | |
|---|---|---|---|---|---|
| | | 0 | 0.5 | 1 | 2 |
| Selectivity of substitution by Na (weight %) | Unreacted product | 98.6 | 1.1 | 1.0 | 0.1 |
| | 1Na compound | 0.0 | 15.5 | 0.7 | 0.6 |
| | 2Na compound | 0.0 | 78.4 | 95.7 | 97.3 |
| | Other impurities | 1.4 | 5.0 | 2.6 | 2.0 |

From Table 1 and Fig. 1, it can be seen that according to the present invention, 78.4 weight% of the 2Na compound was obtained even in a short time of 0.5 hour, and 90% or more of the 2Na compound was obtained in 1 hour of the reaction time which shows that 2 Na compound was obtained from almost all of the raw material phenol compounds.

### [Comparative Example 1]

### <Preparation of Phenol Compound Solution>

The raw material phenol compound was dissolved in tetrahydrofuran (boiling point: about 66°C) at 25°C to prepare a phenol compound solution 2 having a concentration of 0.380 mol/L.

### <Preparation of Sodium Mixed Liquid>

Rodlike sodium was cut into a 5 mm square and mixed with tetrahydrofuran to prepare a sodium mixed liquid 1 having a sodium concentration of 0.836 mol/L.

The average particle diameter (Median diameter D₅₀) of the sodium particles in the sodium mixed liquid 1 was 5 mm and sodium was precipitated in the mixed liquid by allowing the mixed liquid to stand still.

### <Substitution by Sodium and Reaction to Produce Dimethylsilyl Compound>

The sodium mixed liquid 1 (97.48 mL) was placed in a four-necked flask, cooled to 2°C, and the phenol compound solution 2 (97.48 mL) was added dropwise thereto for 2 minutes with stirring the sodium mixed liquid 1 for mixing. After completion of the dropwise addition, the temperature of the mixed liquid in the four-necked flask was increased at 3°C/min to 65°C, and the temperature was maintained at 65°C for 2 hours to perform a substitution reaction by sodium.

The mixing and reaction were performed in a nitrogen atmosphere.

The sodium mixed liquid 1 and the phenol compound solution 2 used in the reaction are such that the amount of sodium is 1.1 equivalents with respect to 1 equivalent of the phenolic hydroxyl group of the raw material phenol compound.

When the liquid chromatography analysis was performed on the mixed liquid at the end of dropwise addition, results were shown in Table 2 (reaction time: 0 hour).

After reaction for 2 hours, dichlorodimethylsilane and tetrahydrofuran were mixed with the obtained reaction product liquid so that the concentration of dichlorodimethylsilane was 0.75 mol/L, and the obtained mixed liquid was added so that the amount of dichlorodimethylsilane was 5 equivalents times the reaction substrate, and the reaction was carried out for 10 minutes with stirring in the nitrogen atmosphere to perform a reaction to produce a dimethylsilyl compound.

The obtained reaction product liquid was subjected to the liquid chromatography analysis, and the results shown in Table 2 (reaction time: 2 hours) were obtained.

In Comparative Example 1, in the mixed liquid of the sodium mixed liquid 1 and the phenol compound solution 2 after the reaction, 6 coarse particles/mL having a major axis of about 3 mm were present.

### <Substitution by Sodium and Reaction to Produce Dimethylsilyl Compound (Reaction Time: 3, 4, 6, 7, 8 Hours)>

The substitution by sodium and the reaction to produce a dimethylsilyl compound were performed in the same manner as described above except that the reaction time for the substitution by sodium was changed from 2 hours to 3 hours, 4 hours, 6 hours, 7 hours, or 8 hours. The results of liquid chromatography analysis at each reaction time are shown in Table 2 (reaction time: 3 hours), Table 2 (reaction time: 4 hours), Table 2 (reaction time: 6 hours), Table 2 (reaction time: 7 hours), or Table 2 (reaction time: 8 hours).

A relationship between the selectivity of substitution by Na and the reaction time based on the liquid chromatography analysis is shown in Fig. 2.

**Table 2**

| | | Reaction time (hr) | | | | | | |
|---|---|---|---|---|---|---|---|---|
| | | 0 | 2 | 3 | 4 | 6 | 7 | 8 |
| Selectivity of substitution by Na (weight %) | Unreacted product | 99.0 | 4.4 | 3.9 | 1.9 | 2.0 | 1.7 | 1.6 |
| | 1Na compound | 0.0 | 23.2 | 18.8 | 17.9 | 15.7 | 11.5 | 11.0 |
| | 2Na compound | 0.0 | 67.3 | 72.2 | 75.3 | 78.1 | 83.0 | 83.6 |
| | Other impurities | 1.0 | 5.1 | 5.1 | 4.9 | 4.2 | 3.8 | 3.8 |

As is clear from Table 2 and Fig. 2, in Comparative Example 1 which is the conventional method, only 67.3 weight% of the target 2Na compound was obtained in the reaction time of 2 hours, and reaction performance was inferior to that in the case of 2 hours of Example 1 even in the reaction for a long time of 8 hours.

Although the present invention has been described in detail with reference to specific examples, it is obvious to those skilled in the art that various changes and modifications may be made without departing from the scope of the present invention.

## Claims

1. A method for producing a sodium salt of a phenol compound, comprising
a step of mixing a phenol compound solution in which a phenol compound having two or more phenolic hydroxyl groups is dissolved and a sodium dispersion liquid in which sodium particles are dispersed.

2. The method for producing the sodium salt of the phenol compound according to claim 1,
wherein the phenol compound solution is obtained by dissolving the phenol compound in a polar solvent.

3. The method for producing the sodium salt of the phenol compound according to claim 2,
wherein the polar solvent contains tetrahydrofuran.

4. The method for producing the sodium salt of the phenol compound according to any one of claims 1 to 3,
wherein the sodium dispersion liquid is obtained by dispersing the sodium particles in an organic solvent.

5. The method for producing the sodium salt of the phenol compound according to claim 4,
wherein the organic solvent contains paraffin.

6. The method for producing the sodium salt of the phenol compound according to claim 4 or 5,
wherein the organic solvent further contains tetrahydrofuran.

7. The method for producing the sodium salt of the phenol compound according to any one of claims 1 to 6,
wherein an average particle diameter (D₅₀) of the sodium particles in the sodium dispersion liquid is 70 µm or less.

8. The method for producing the sodium salt of the phenol compound according to any one of claims 1 to 7,
wherein the sodium dispersion liquid and the phenol compound solution are mixed under such a condition that the sodium particles are not associated with each other.

9. The method for producing the sodium salt of the phenol compound according to any one of claims 1 to 8,
wherein the phenol compound solution and the sodium dispersion liquid are mixed at a temperature lower than a melting point of sodium.

10. The method for producing the sodium salt of the phenol compound according to claim 9,
wherein the phenol compound solution and the sodium dispersion liquid are mixed at a temperature lower than a lowest boiling point among boiling points of solvents contained in the phenol compound solution and the sodium dispersion liquid.

11. The method for producing the sodium salt of the phenol compound according to any one of claims 1 to 10,
wherein the number of sodium particles having a major axis of more than 70 µm present in a mixed liquid of the phenol compound solution and the sodium dispersion liquid is 5 particles/mL or less.

12. The method for producing the sodium salt of the phenol compound according to any one of claims 1 to 11,
wherein the phenol compound having two or more phenolic hydroxyl groups is dihydroxybiphenyl.

13. The method for producing the sodium salt of the phenol compound according to claim 12,
wherein the dihydroxybiphenyl is a compound represented by the following Formula (1): 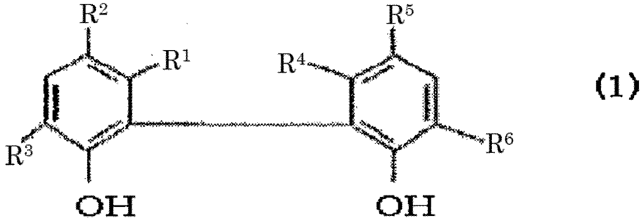
wherein each of R¹ and R⁴ independently represents a hydrogen atom, an alkyl group having 1 to 12 carbon atoms, a cycloalkyl group having from 3 to 12 carbon atoms, an alkoxy group having from 1 to 12 carbon atoms, a silyl group having from 1 to 12 carbon atoms, a siloxy group having from 1 to 12 carbon atoms, or a halogen atom, and
each of R², R³, R⁵, and R⁶ each independently represents an alkyl group having from 1 to 20 carbon atoms, a cycloalkyl group having from 3 to 20 carbon atoms, an alkoxy group having from 1 to 20 carbon atoms, an aryl group having from 6 to 20 carbon atoms, a silyl group having from 1 to 20 carbon atoms, or a siloxy group having from 1 to 20 carbon atoms.

14. The method for producing the sodium salt of the phenol compound according to claim 13,
wherein the compound represented by Formula (1) is 3,3',5,5'-tetratertiary-butyl-6,6' -dimethyl-2,2' -biphenol.

15. A method for producing a bisphosphite compound, comprising
producing a sodium salt of a phenol compound by the method according to claim 12, and
further allowing the produced sodium salt of the phenol compound to react with a phosphorus compound to produce the bisphosphite compound.

16. The method for producing a bisphosphite compound according to claim 15,
wherein the bisphosphite compound is represented by the following Formula (2): 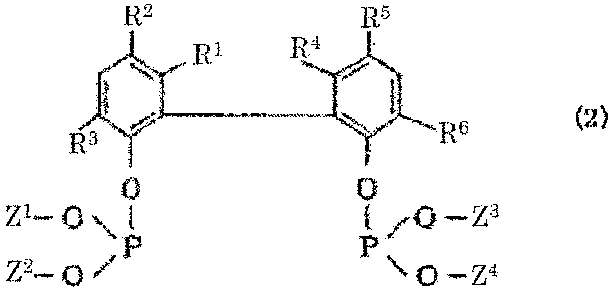
wherein each of R¹ and R⁴ independently represents a hydrogen atom, an alkyl group having from 1 to 12 carbon atoms, a cycloalkyl group having from 3 to 12 carbon atoms, an alkoxy group having from 1 to 12 carbon atoms, a silyl group having from 1 to 12 carbon atoms, a siloxy group having from 1 to 12 carbon atoms, or a halogen atom,
each of R², R³, R⁵, and R⁶ independently represents an alkyl group having from 1 to 20 carbon atoms, a cycloalkyl group having from 3 to 20 carbon atoms, an alkoxy group having from 1 to 20 carbon atoms, an aryl group having from 6 to 20 carbon atoms, a silyl group having from 1 to 20 carbon atoms, or a siloxy group having from 1 to 20 carbon atoms, and
each of Z¹, Z², Z³, and Z⁴ independently represents an alkyl group having from 1 to 20 carbon atoms, a cycloalkyl group having from 3 to 20 carbon atoms, a substituted or unsubstituted aryl group having from 6 to 20 carbon atoms, an aralkyl group having from 7 to 20 carbon atoms, or a heteroaryl group having from 1 to 20 carbon atoms; and
wherein the dihydroxybiphenyl used for producing the sodium salt of the phenol compound is a compound represented by Formula (1): 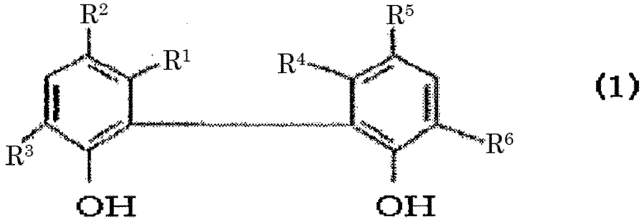
wherein R¹ to R⁶ are as defined above.

17. The method for producing a bisphosphite compound according to claim 16, wherein the bisphosphite compound is represented by the following Formula (3):

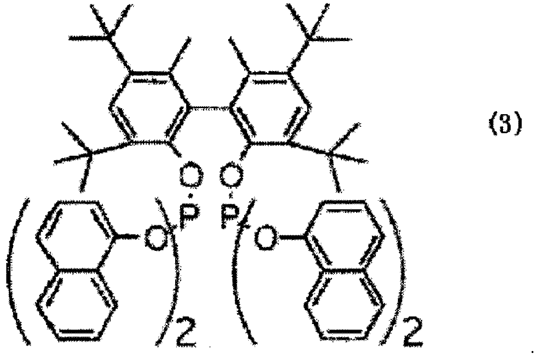

## Patentansprüche

1. Verfahren zur Herstellung eines Natriumsalzes einer Phenolverbindung, umfassend:
einen Schritt zum Mischen einer Phenolverbindungslösung, in der eine Phenolverbindung mit zwei oder mehr phenolischen Hydroxygruppen gelöst ist, und einer Natriumdispersionsflüssigkeit, in der Natriumpartikel dispergiert sind.

2. Verfahren zur Herstellung des Natriumsalzes der Phenolverbindung gemäß Anspruch 1,
wobei die Phenolverbindungslösung durch Lösen der Phenolverbindung in einem polaren Lösungsmittel erhalten wird.

3. Verfahren zur Herstellung des Natriumsalzes der Phenolverbindung gemäß Anspruch 2,
wobei das polare Lösungsmittel Tetrahydrofuran enthält.

4. Verfahren zur Herstellung des Natriumsalzes der Phenolverbindung gemäß einem der Ansprüche 1 bis 3,
wobei die Natriumdispersionsflüssigkeit durch Dispergieren der Natriumpartikel in einem organischen Lösungsmittel erhalten wird.

5. Verfahren zur Herstellung des Natriumsalzes der Phenolverbindung gemäß Anspruch 4,
wobei das organische Lösungsmittel Paraffin enthält.

6. Verfahren zur Herstellung des Natriumsalzes der Phenolverbindung gemäß Anspruch 4 oder 5,
wobei das organische Lösungsmittel ferner Tetrahydrofuran enthält.

7. Verfahren zur Herstellung des Natriumsalzes der Phenolverbindung gemäß einem der Ansprüche 1 bis 6,
wobei ein durchschnittlicher Partikeldurchmesser (D₅₀) der Natriumpartikel in der Natriumdispersionsflüssigkeit 70 µm oder weniger beträgt.

8. Verfahren zur Herstellung des Natriumsalzes der Phenolverbindung gemäß einem der Ansprüche 1 bis 7,
wobei die Natriumdispersionsflüssigkeit und die Phenolverbindungslösung unter einer solchen Bedingung gemischt werden, dass die Natriumpartikel nicht miteinander assoziieren.

9. Verfahren zur Herstellung des Natriumsalzes der Phenolverbindung gemäß einem der Ansprüche 1 bis 8,
wobei die Phenolverbindungslösung und die Natriumdispersionsflüssigkeit bei einer Temperatur gemischt werden, die niedriger als ein Schmelzpunkt von Natrium ist.

10. Verfahren zur Herstellung des Natriumsalzes der Phenolverbindung gemäß Anspruch 9,
wobei die Phenolverbindungslösung und die Natriumdispersionsflüssigkeit bei einer Temperatur gemischt werden, die niedriger als ein niedrigster Siedepunkt unter den Siedepunkten der Lösungsmittel ist, die in der Phenolverbindungslösung und der Natriumdispersionsflüssigkeit enthalten sind.

11. Verfahren zur Herstellung des Natriumsalzes der Phenolverbindung gemäß einem der Ansprüche 1 bis 10,
wobei die Anzahl von Natriumpartikeln mit einer Hauptachse von mehr als 70 µm, die in einem Flüssigkeitsgemisch aus der Phenolverbindungslösung und der Natriumdispersionsflüssigkeit vorhanden sind, 5 Partikel/mL oder weniger beträgt.

12. Verfahren zur Herstellung des Natriumsalzes der Phenolverbindung gemäß einem der Ansprüche 1 bis 11,
wobei die Phenolverbindung mit zwei oder mehr phenolischen Hydroxylgruppen Dihydroxybiphenyl ist.

13. Verfahren zur Herstellung des Natriumsalzes der Phenolverbindung gemäß Anspruch 12,
wobei das Dihydroxybiphenyl eine durch die folgende Formel (1) dargestellte Verbindung ist: 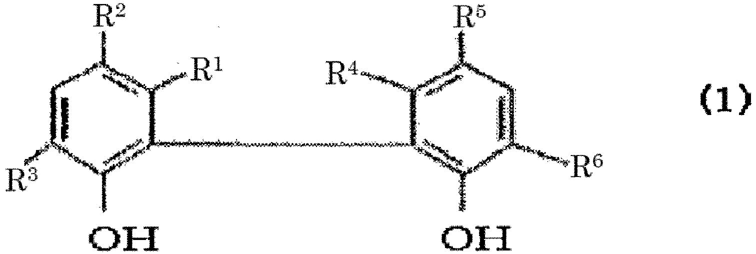
wobei jedes von R¹ und R⁴ unabhängig ein Wasserstoffatom, eine Alkylgruppe mit 1 bis 12 Kohlenstoffatomen, eine Cycloalkylgruppe mit 3 bis 12 Kohlenstoffatomen, eine Alkoxygruppe mit 1 bis 12 Kohlenstoffatomen, eine Silylgruppe mit 1 bis 12 Kohlenstoffatomen, eine Siloxygruppe mit 1 bis 12 Kohlenstoffatomen oder ein Halogenatom darstellen und
jedes von R², R³, R⁵ und R⁶ jeweils unabhängig eine Alkylgruppe mit 1 bis 20 Kohlenstoffatomen, eine Cycloalkylgruppe mit 3 bis 20 Kohlenstoffatomen, eine Alkoxygruppe mit 1 bis 20 Kohlenstoffatomen, eine Arylgruppe mit 6 bis 20 Kohlenstoffatomen, eine Silylgruppe mit 1 bis 20 Kohlenstoffatomen oder eine Siloxygruppe mit 1 bis 20 Kohlenstoffatomen darstellen.

14. Verfahren zur Herstellung des Natriumsalzes der Phenolverbindung gemäß Anspruch 13,
wobei die durch Formel (1) dargestellte Verbindung 3,3',5,5'-tertiäres-Butyl-6,6'-dimethyl-2-2'-biphenol ist.

15. Verfahren zur Herstellung eine Bisphosphitverbindung, umfassend
Herstellen eines Natriumsalzes einer Phenolverbindung durch das Verfahren gemäß Anspruch 12 und
ferner Reagierenlassen des hergestellten Natriumsalzes der Phenolverbindung mit einer Phosphorverbindung, um die Bisphosphitverbindung herzustellen.

16. Verfahren zur Herstellung einer Bisphosphitverbindung gemäß Anspruch 15,
wobei die Bisphosphitverbindung durch die folgende Formel (2) dargestellt ist: 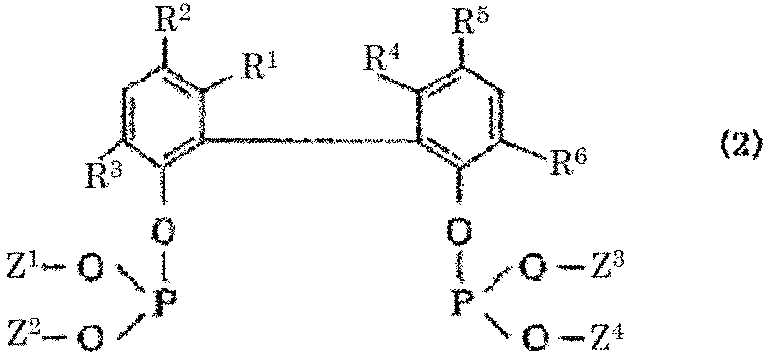
wobei jedes von R¹ und R⁴ unabhängig ein Wasserstoffatom, eine Alkylgruppe mit 1 bis 12 Kohlenstoffatomen, eine Cycloalkylgruppe mit 3 bis 12 Kohlenstoffatomen, eine Alkoxygruppe mit 1 bis 12 Kohlenstoffatomen, eine Silylgruppe mit 1 bis 12 Kohlenstoffatomen, eine Siloxygruppe mit 1 bis 12 Kohlenstoffatomen oder ein Halogenatom darstellen und
jedes von R², R³, R⁵ und R⁶ unabhängig eine Alkylgruppe mit 1 bis 20 Kohlenstoffatomen, eine Cycloalkylgruppe mit 3 bis 20 Kohlenstoffatomen, eine Alkoxygruppe mit 1 bis 20 Kohlenstoffatomen, eine Arylgruppe mit 6 bis 20 Kohlenstoffatomen, eine Silylgruppe mit 1 bis 20 Kohlenstoffatomen oder eine Siloxygruppe mit 1 bis 20 Kohlenstoffatomen darstellen und
jedes von Z¹, Z², Z³ und Z⁴ unabhängig eine Alkylgruppe mit 1 bis 20 Kohlenstoffatomen, eine Cycloalkylgruppe mit 3 bis 20 Kohlenstoffatomen, eine substituierte oder unsubstituierte Arylgruppe mit 6 bis 20 Kohlenstoffatomen, eine Aralkylgruppe mit 7 bis 20 Kohlenstoffatomen oder eine Heteroarylgruppe mit 1 bis 20 Kohlenstoffatomen darstellen, und
wobei das Dihydroxybiphenyl, das zur Herstellung des Natriumsalzes der Phenolverbindung verwendet wird, eine durch Formel (1) dargestellte Verbindung ist: 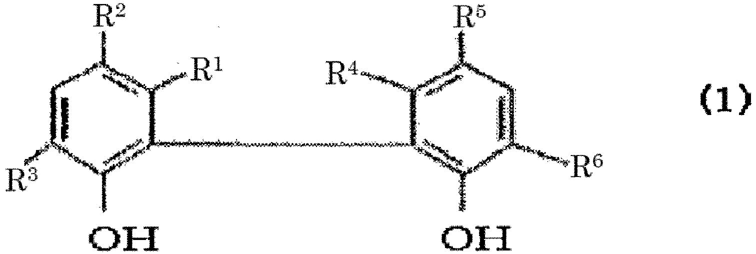
wobei R¹ bis R⁶ wie oben definiert sind.

17. Verfahren zur Herstellung einer Bisphosphitverbindung gemäß Anspruch 16,
wobei die Bisphosphitverbindung durch die folgende Formel (3) dargestellt ist:

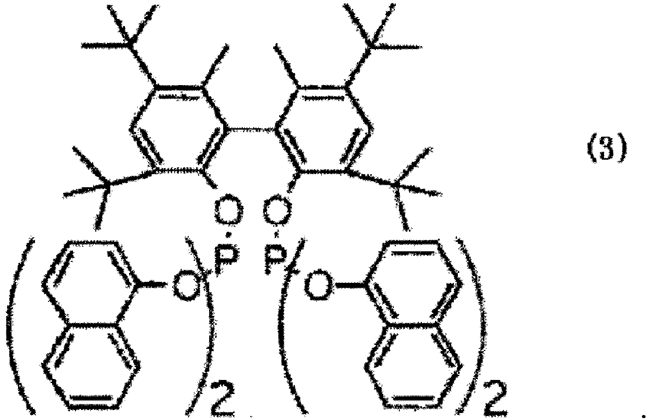

## Revendications

1. Procédé de production un sel de sodium d'un composé phénolique, comprenant
une étape consistant à mélanger une solution de composé phénolique dans laquelle un composé phénolique présentant deux ou plusieurs groupes hydroxyle phénoliques est dissout et un liquide de dispersion de sodium dans lequel des particules de sodium sont dispersées.

2. Procédé de production du sel de sodium du composé phénolique selon la revendication 1,
dans lequel la solution de composé phénolique est obtenue en dissolvant le composé phénolique dans un solvant polaire.

3. Procédé de production du sel de sodium du composé phénolique selon la revendication 2,
dans lequel le solvant polaire contient du tétrahydrofuranne.

4. Procédé de production du sel de sodium du composé phénolique selon l'une quelconque des revendications 1 à 3,
dans lequel le liquide de dispersion de sodium est obtenu en dispersant les particules de sodium dans un solvant organique.

5. Procédé de production du sel de sodium du composé phénolique selon la revendication 4,
dans lequel le solvant organique contient de la paraffine.

6. Procédé de production du sel de sodium du composé phénolique selon la revendication 4 ou la revendication 5,
dans lequel le solvant organique contient en outre du tétrahydrofuranne.

7. Procédé de production du sel de sodium du composé phénolique selon l'une quelconque des revendications 1 à 6,
dans lequel le diamètre moyen de particules (D₅₀) des particules de sodium dans le liquide de dispersion de sodium est inférieur ou égal à 70 µm.

8. Procédé de production du sel de sodium du composé phénolique selon l'une quelconque des revendications 1 à 7,
dans lequel le liquide de dispersion de sodium et la solution de composé phénolique sont mélangés dans des conditions telles que les particules de sodium ne sont pas associées les unes aux autres.

9. Procédé de production du sel de sodium du composé phénolique selon l'une quelconque des revendications 1 à 8,
dans lequel la solution de composé phénolique et le liquide de dispersion de sodium sont mélangés à une température inférieure au point de fusion du sodium.

10. Procédé de production du sel de sodium du composé phénolique selon la revendication 9,
dans lequel la solution de composé phénolique et le liquide de dispersion de sodium sont mélangés à une température inférieure au point d'ébullition le plus bas parmi les points d'ébullition de solvants contenus dans la solution de composé phénolique et le liquide de dispersion de sodium.

11. Procédé de production du sel de sodium du composé phénolique selon l'une quelconque des revendications 1 à 10,
dans lequel le nombre de particules de sodium ayant un grand axe supérieur à 70 µm présentes dans un liquide mélangé de la solution de composé phénolique et du liquide de dispersion de sodium est inférieur ou égal à 5 particules/mL.

12. Procédé de production du sel de sodium du composé phénolique selon l'une quelconque des revendications 1 à 11,
dans lequel le composé phénolique ayant deux ou plusieurs groupes hydroxyle phénoliques est du dihydroxybiphényle.

13. Procédé de production du sel de sodium du composé phénolique selon la revendication 12,
dans lequel le dihydroxybiphényle est un composé représenté par la formule suivante (1) : 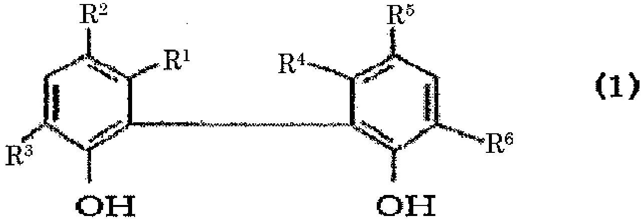
dans lequel chacun de R¹ et R⁴ représente indépendamment un atome d'hydrogène, un groupe alkyle ayant de 1 à 12 atomes de carbone, un groupe cycloalkyle présentant de 3 à 12 atomes de carbone, un groupe alcoxy ayant de 1 à 12 atomes de carbone, un groupe silyle ayant de 1 à 12 atomes de carbone, un groupe siloxy ayant de 1 à 12 atomes de carbone, ou un atome d'halogène, et
chacun de R², R³, R⁵ et R⁶ représente chacun indépendamment un groupe alkyle ayant de 1 à 20 atomes de carbone, un groupe cycloalkyle présentant de 3 à 20 atomes de carbone, un groupe alcoxy ayant de 1 à 20 atomes de carbone, un groupe aryle ayant de 6 à 20 atomes de carbone, un groupe silyle ayant de 1 à 20 atomes de carbone, ou un groupe siloxy ayant de 1 à 20 atomes de carbone.

14. Procédé de production du sel de sodium du composé phénolique selon la revendication 13,
dans lequel le composé représenté par la formule (1) est 3,3',5,5'-tétratertiaire-butyl-6,6'-diméthyl-2,2'-biphénol.

15. Procédé de production un composé bisphosphite, comprenant
produire un sel de sodium d'un composé phénolique par le procédé selon la revendication 12, et
faire réagir en outre le sel de sodium produit du composé phénolique avec un composé de phosphore pour produire le composé bisphosphite.

16. Procédé de production d'un composé bisphosphite selon la revendication 15, dans lequel le composé bisphosphite est représenté par la formule suivante (2) : 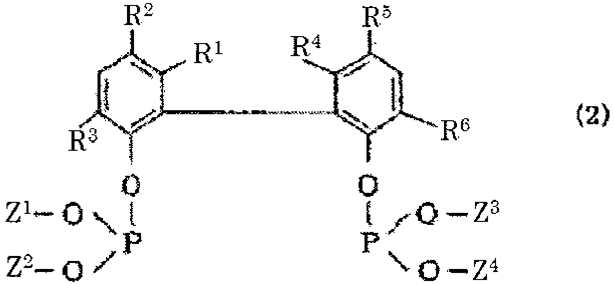
dans lequel chacun de R¹ et R⁴ représente indépendamment un atome d'hydrogène, un groupe alkyle ayant de 1 à 12 atomes de carbone, un groupe cycloalkyle présentant de 3 à 12 atomes de carbone, un groupe alcoxy ayant de 1 à 12 atomes de carbone, un groupe silyle ayant de 1 à 12 atomes de carbone, un groupe siloxy ayant de 1 à 12 atomes de carbone, ou un atome d'halogène,
chacun de R², R³, R⁵ et R⁶ représente indépendamment un groupe alkyle ayant de 1 à 20 atomes de carbone, un groupe cycloalkyle présentant de 3 à 20 atomes de carbone, un groupe alcoxy ayant de 1 à 20 atomes de carbone, un groupe aryle ayant de 6 à 20 atomes de carbone, un groupe silyle ayant de 1 à 20 atomes de carbone, ou un groupe siloxy ayant de 1 à 20 atomes de carbone, et
chacun de Z¹, Z², Z³ et Z⁴ représente indépendamment un groupe alkyle ayant de 1 à 20 atomes de carbone, un groupe cycloalkyle présentant de 3 à 20 atomes de carbone, un groupe aryle substitué ou non substitué ayant de 6 à 20 atomes de carbone, un groupe aralkyle ayant de 7 à 20 atomes de carbone, ou un groupe hétéroaryle ayant de 1 à 20 atomes de carbone ; et
dans lequel le dihydroxybiphényle utilisé pour produire le sel de sodium du composé phénolique est un composé représenté par la formule (1) : 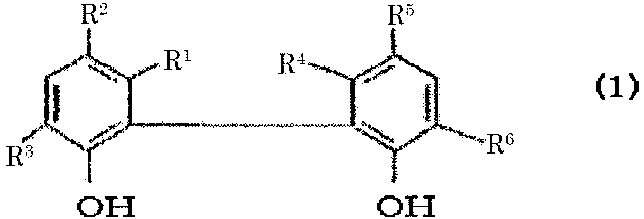
dans lequel R¹ à R⁶ sont tels que précédemment défini.

17. Procédé de production d'un composé bisphosphite selon la revendication 16, dans lequel le composé bisphosphite est représenté par la formule suivante (3) :
